# EUROPEAN PATENT APPLICATION

(11) **EP 4 317 400 A1**
(43) Date of publication of application: **07.02.2024**
(21) Application number: 21933250.9
(22) Date of filing: 08.12.2021
(51) Int. Cl.: C12M 1/12, C12M 1/26, C12Q 1/04, C12Q 1/22, C12Q 1/24, G01N 1/10

(54) **CENTRIFUGAL FILTRATION CARTRIDGE AND MICROBIAL TEST METHOD**

(30) Priority: 24.03.2021 JP 2021050339
(71) Applicant: HITACHI HIGH-TECH CORPORATION, Tokyo 105-6409 (JP)
(72) Inventor: KAWABE Shunsuke, Tokyo 100-8280 (JP); ISHIMARU Masako, Tokyo 100-8280 (JP)
(74) Representative: MERH-IP Matias Erny Reichl Hoffmann Patentanwälte PartG mbB
(86) International application number: PCT/JP2021/045143
(87) International publication number: WO 2022/201657

(57) **Abstract**

Provided is a technique for reducing false positives and false negatives in a microbial test. A centrifugal filtration cartridge of the present disclosure includes: a filter cup including a filter that captures a microorganism and a nozzle that discharges a liquid that has passed through the filter; and a contamination prevention box connectable to the filter cup and internally storing a measurement container configured to store the liquid discharged from the nozzle. The contamination prevention box includes a partition wall disposed between the filter cup and an opening surface of the measurement container. The partition wall has an opening through which the nozzle can pass. The nozzle has a length in which a tip of the nozzle is located below a lower end of the opening and enters inside of the measurement container when the contamination prevention box is connected to the filter cup.

## Description

### Technical Field

The present disclosure relates to a centrifugal filtration cartridge and a microbial test method.

### Background Art

In order to ensure cleanliness of a production facility, or in order to ensure sterility of water (for example, pharmaceutical water) used for production or a product itself, a microbial test or a sterility test is performed. In general, a membrane filter method (MF method) using culture of bacteria is adopted as a method of the microbial test or the sterility test. The MF method is a method in which a collected sample is filtered through a membrane filter, then the membrane filter is placed on an agar medium, and cultured in a thermostat for up to 14 days, and the number of colonies of grown bacteria is visually counted. However, even fast growing bacteria require a period of at least several days to reach visual detection of colonies. In addition, it has been revealed by progress of microorganism research in recent years that there are bacteria that are difficult to detect and quantify only by culturing since a conventional culture medium has low colony-forming ability. As a means for solving these problems, expectations are placed on a rapid microbial test technique using various measurement principles different from the conventional MF method using culture.

Rapid microbial test methods are roughly divided into direct detection methods and indirect detection methods. The former includes solid phase cytometry, flow cytometry, and the like. The latter includes immunological methods, nucleic acid amplification methods, bioluminescence, fluorescence (staining) methods, and the like. For example, there is an ATP bioluminescence method (hereinafter referred to as the ATP method) using adenosine triphosphate (ATP) contained in bacteria. This method is a method in which ATP contained in bacteria is extracted and reacted with luciferase as an enzyme to cause bioluminescence, and an amount of luminescence thereof is measured to estimate an amount of bacteria. In the ATP method, the presence of ATP molecules contained per bacterium can be detected as several tens of thousands of photons. Therefore, it is possible to perform high sensitivity detection at a level of one bacterium, and the ATP method is expected as the rapid microbial test method.

Performance to be prioritized in performance required for speeding up the sterility test is detection sensitivity of bacteria, whereas a probability of false positives increases as the detection sensitivity is improved. In the ATP method, in order to detect an extremely small amount (several pieces level) of bacteria present in a specimen with high sensitivity and high accuracy, contamination of ATP and bacteria present in the surrounding environment must be suppressed while performance of a luminescence measurement device is high sensitivity.

PTL 1 describes a filtering member and a filtering method for reducing sample contamination from the surrounding environment in order to perform luminescence detection on bacteria-derived ATP with high sensitivity.

### Citation List

### Patent Literature

PTL 1: JP 2017-225448 A

### Summary of Invention

### Technical Problem

In general, in the microbial test or the sterility test, a measurement device for performing the test is used. It is necessary to extract a target substance (for example, bacteria-derived ATP) from a test sample and then transfer an aqueous solution containing the target substance to a measurement container dedicated to the measurement device. However, in the middle of the transfer operation, there are risks of loss of the aqueous solution containing the target substance to the outside of the measurement container, or mixing of a substance that causes inhibition of measurement (for example, bacteria, ATP, or the like present in the surrounding environment). The loss of the target substance or the mixing of the substance inhibiting the measurement causes false positive and false negative of the test.

For example, in the microbial test using the ATP method, the test sample is filtered with a filter and ATP is extracted from a bacterium trapped by the filter. Then it is necessary to transfer an aqueous solution containing bacteria-derived ATP to a measurement tube dedicated to the measurement device. At this time, if the aqueous solution leaks out of the measurement tube or an inhibitory substance (for example, ATP derived from the environment) is mixed from the surrounding environment, false positives and false negatives are caused.

In particular, in the sterility test, contamination from the surrounding environment must be strictly prevented. In the sterility test, a centrifuge is often used for filtration and movement of the test sample in order to ensure sealability of the test sample. However, in the liquid feeding by a centrifugal force, a liquid feeding direction is not accurately determined as compared with other liquid feeding methods (for example, aspiration filtration by an aspiration pump). Therefore, the test sample may come into contact with an unexpected unnecessary portion. Also in the ATP method, when the specimen is delivered by centrifugal filtration and moved to the measurement tube, the aqueous solution may come into contact with a portion other than a filtration filter and the measurement tube, and ATP from the surrounding environment may contaminate. The mixing of ATP from the surrounding environment causes false positives.

In the sterility test by the ATP method, in order to detect an extremely small amount of bacteria, ATP may be extracted with a small amount of extraction liquid (for example, several tens µL) after filtering the test sample with the filter. This is because ATP extracted from a bacterium is concentrated to as high concentration as possible to measure luminescence. However, as the amount of the extraction liquid decreases, it becomes more difficult to accurately transfer the test sample without losing the test sample when the test sample is delivered from the filter to the measurement tube by centrifugal filtration. For example, the test sample may remain on a bottom surface of the filter and in unevenness of a flow path from a filtrate discharge portion to the measurement tube. The loss of the test sample causes false negatives.

Thus, the present disclosure provides a technique for reducing false positives and false negatives in a microbial test.

### Solution to Problem

In order to solve the above problems, a centrifugal filtration cartridge of the present disclosure includes: a filter cup including a filter that traps a microorganism and a nozzle that discharges a liquid that has passed through the filter; and a contamination prevention box connectable to the filter cup and internally storing a measurement container configured to store the liquid discharged from the nozzle. The contamination prevention box includes a partition wall disposed between the filter cup and an opening surface of the measurement container. The partition wall has an opening through which the nozzle can pass. The nozzle has a length in which a tip of the nozzle is located below a lower end of the opening and enters inside of the measurement container when the contamination prevention box is connected to the filter cup.

Other features of the disclosure will be apparent from the description and the accompanying drawings of this specification. In addition, embodiments of the present disclosure are achieved and realized by elements, combinations of various elements, the following detailed description, and the attached claims. It is necessary to understand that the description of this specification is given only as a typical example, and does not limit the scope of claims or applications of the present disclosure.

### Advantageous Effects of Invention

According to the technology of the present disclosure, it is possible to reduce false positives and false negatives in a microbial test. Objects, configurations, and effects other than those described above will be apparent through the explanation on the following embodiments.

### Brief Description of Drawings

FIG. 1 is a six-sided view illustrating an appearance of a centrifugal filtration cartridge according to a first embodiment in a first state.
FIG. 2 is a perspective view illustrating the appearance of the centrifugal filtration cartridge according to the first embodiment in the first state.
FIG. 3 is a reference view illustrating a portion made of a light-transmissive material of the centrifugal filtration cartridge according to the first embodiment in the first state.
FIG. 4 is a cross-sectional view taken along line I-I of the centrifugal filtration cartridge according to the first embodiment in the first state.
FIG. 5 is a six-sided view illustrating an appearance of a contamination prevention box of the centrifugal filtration cartridge according to the first embodiment.
FIG. 6 is a perspective view illustrating the appearance of the contamination prevention box of the centrifugal filtration cartridge according to the first embodiment.
FIG. 7 is a reference view illustrating a portion made of a light-transmissive material of the contamination prevention box of the centrifugal filtration cartridge according to the first embodiment.
FIG. 8 is a cross-sectional view taken along line V-V of the contamination prevention box of the centrifugal filtration cartridge according to the first embodiment.
FIG. 9 is a six-sided view illustrating an appearance of the centrifugal filtration cartridge according to the first embodiment in a second state.
FIG. 10 is a perspective view illustrating the appearance of the centrifugal filtration cartridge according to the first embodiment in the second state.
FIG. 11 is a reference view illustrating a portion made of a light-transmissive material of the centrifugal filtration cartridge according to the first embodiment in the second state.
FIG. 12 is a cross-sectional view taken along line IX-IX of the centrifugal filtration cartridge according to the first embodiment in the second state.
FIG. 13 is a flowchart of a sterility test method using the centrifugal filtration cartridge according to the first embodiment.
FIG. 14A is a schematic cross-sectional view illustrating a state of the centrifugal filtration cartridge in step S101 of the sterility test method according to the first embodiment.
FIG. 14B is a schematic cross-sectional view illustrating a state of the centrifugal filtration cartridge in step S102 of the sterility test method according to the first embodiment.
FIG. 14C is a schematic cross-sectional view illustrating a state of the centrifugal filtration cartridge in step S103 of the sterility test method according to the first embodiment.
FIG. 14D is a schematic cross-sectional view illustrating a state of the centrifugal filtration cartridge in step S104 of the sterility test method according to the first embodiment.
FIG. 14E is a schematic cross-sectional view illustrating a state of the centrifugal filtration cartridge in step S105 of the sterility test method according to the first embodiment.
FIG. 14F is a schematic cross-sectional view illustrating a state of the centrifugal filtration cartridge in step S106 of the sterility test method according to the first embodiment.
FIG. 14G is a schematic cross-sectional view illustrating a state of the centrifugal filtration cartridge in step S107 of the sterility test method according to the first embodiment.
FIG. 14H is a schematic cross-sectional view illustrating a state of the centrifugal filtration cartridge in step S108 of the sterility test method according to the first embodiment.
FIG. 14I is a schematic cross-sectional view illustrating a state of the centrifugal filtration cartridge in step S109 of the sterility test method according to the first embodiment.
FIG. 15 is a schematic cross-sectional view illustrating a centrifugal filtration cartridge according to a second embodiment.
FIG. 16 is a schematic cross-sectional view illustrating a centrifugal filtration cartridge according to a third embodiment.

### Description of Embodiments

### [First Embodiment]

### <Configuration Example of First State of Centrifugal Filtration Cartridge>

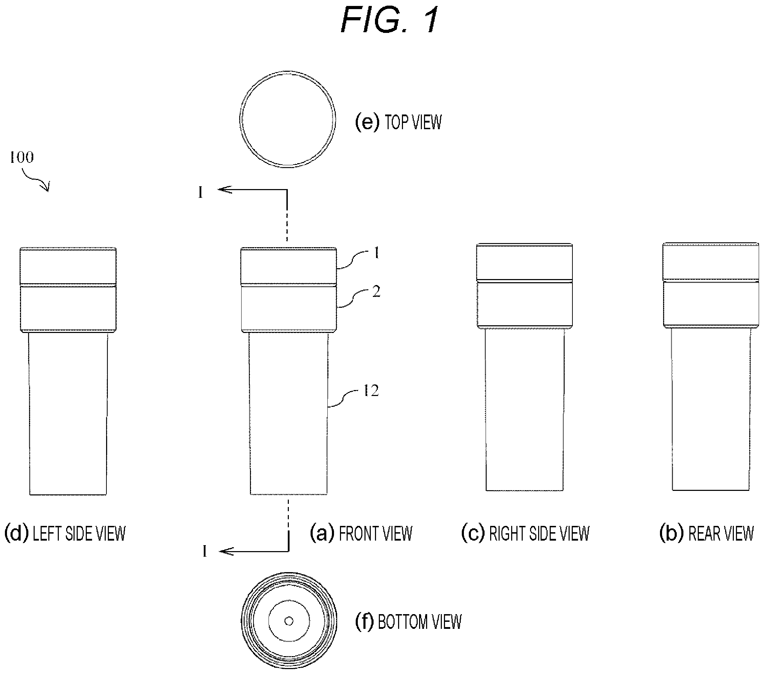

FIG. 1 is a six-sided view illustrating an appearance of a centrifugal filtration cartridge 100 according to a first embodiment in a first state. FIG. 1(a) is a front view. FIG. 1(b) is a rear view. FIG. 1(c) is a right side view. FIG. 1(d) is a left side view. FIG. 1(e) is a top view. FIG. 1(f) is a bottom view. As illustrated in FIG. 1, the centrifugal filtration cartridge 100 in the first state includes a lidded holder 2 and a waste liquid container 12. The lidded holder 2 is provided with an openable lid 1. The lidded holder 2 and the waste liquid container 12 are configured to be detachable, which will be described later in detail.

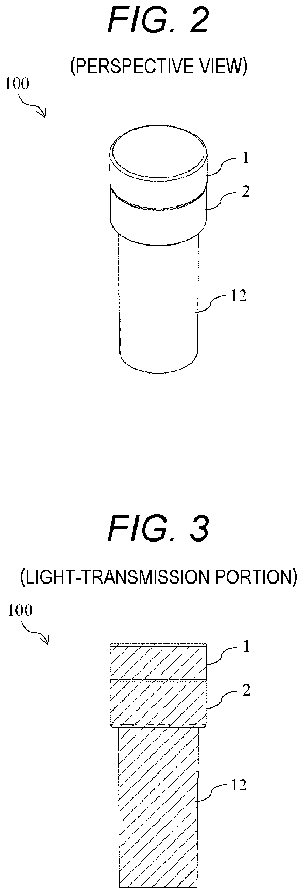

FIG. 2 is a perspective view of the centrifugal filtration cartridge 100 in the first state. As illustrated in FIG. 2, the lidded holder 2 and the waste liquid container 12 each have a substantially cylindrical shape. An outer diameter of the waste liquid container 12 is smaller than an outer diameter of the lidded holder 2.

FIG. 3 is a reference view illustrating a portion made of a light-transmissive material of the centrifugal filtration cartridge 100 in the first state. A hatched portion in FIG. 3, that is, the lid 1, the lidded holder 2, and the waste liquid container 12 can be made of a material (resin or glass) that transmits light.

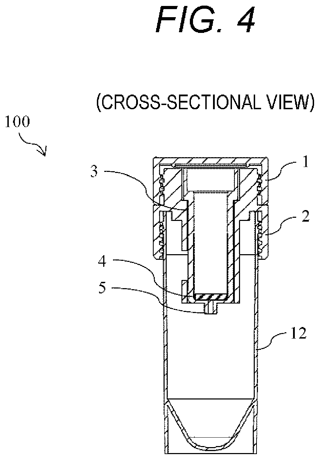

FIG. 4 is a cross-sectional view taken along line I-I of the centrifugal filtration cartridge 100 in the first state. As illustrated in FIG. 4, the lidded holder 2 includes a filter cup 3. The filter cup 3 has a substantially tubular shape. A test sample (solution) is introduced into the filter cup 3. An upper end of the filter cup 3 is at the same height as an upper end of the lidded holder 2. The filter cup 3 has a filter 4 disposed at an inner bottom portion. A nozzle 5 is provided at a lower end of the filter cup 3. A liquid that has passed through the filter 4 is discharged from the nozzle 5. By simply providing an opening in the filter cup 3 instead of providing the nozzle 5, a filtrate obtained by filtering the test sample with the filter 4 can be discharged from the filter cup 3.

The filter 4 may be any filter as long as it can filter the test sample introduced into the filter cup 3 and trap microorganisms (bacteria, fungi, archaea, and the like) to be tested, and for example, a membrane filter can be used. Examples of the material of the filter 4 include cellulose mixed ester, PTFE, PVDF (Polyvinylidene Fluoride), and polycarbonate.

A pore size of the filter 4 can be appropriately selected according to the size of the microorganism to be tested and various conditions of the microbial test, and can be, for example, 0.20 um or more. Specifically, examples of the filter 4 include membrane filters having pore sizes of 0.22 um, 0.45 um, and the like.

The test sample is introduced into the filter cup 3, the lid 1 is sealed, the centrifugal filtration cartridge 100 is set in a centrifuge in a state where the lidded holder 2 and the waste liquid container 12 are connected, and centrifugation is performed. Consequently, the test sample added to the filter cup 3 passes through the filter 4 and is filtered, and the filtrate (waste liquid) is collected in the waste liquid container 12.

The lidded holder 2 has two portions having different outer diameters. The outer diameter of an upper portion of the lidded holder 2 is smaller than the outer diameter of a lower portion. The outer diameter of the lid 1 and the outer diameter of the upper portion of the lidded holder 2 are substantially equal. An inner diameter of the lower portion of the lidded holder 2 is substantially equal to the outer diameter of the waste liquid container 12. The lidded holder 2 and the waste liquid container 12 are provided with a screw groove. The waste liquid container 12 is screw fitted (connected) with the lidded holder 2. The connection method between the lidded holder 2 and the waste liquid container 12 is not limited to the screw type, and it is sufficient that a fixing strength that can withstand centrifugation by a centrifuge can be obtained. For example, the lidded holder 2 and the waste liquid container 12 may be configured to be connectable simply by fitting.

In the example illustrated in FIG. 4, the lid 1 is configured to be detachable from the upper portion of the lidded holder 2 by a screw type. However, the lid 1 may be a hinge mechanism.

### <Configuration Example of Contamination Prevention Box>

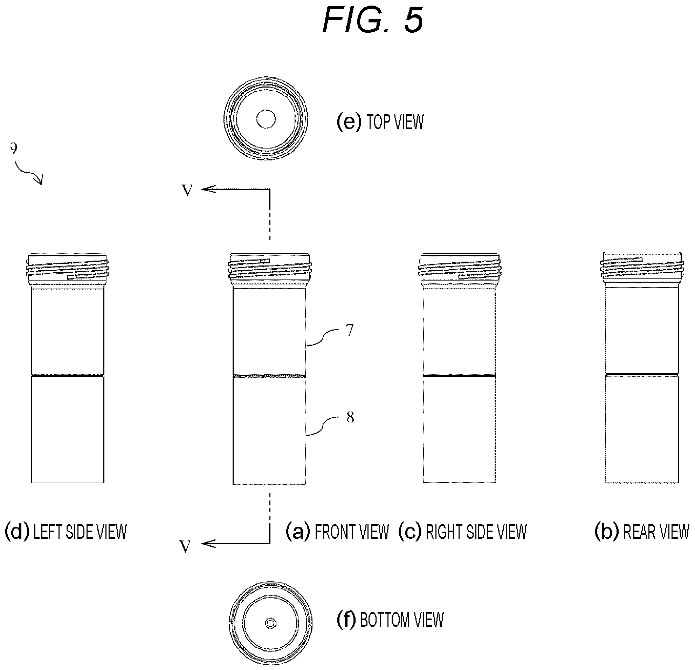

FIG. 5 is a six-sided view illustrating an appearance of the contamination prevention box 9 of the centrifugal filtration cartridge 100. FIG. 5(a) is a front view. FIG. 5(b) is a rear view. FIG. 5(c) is a right side view. FIG. 5(d) is a left side view. FIG. 5(e) is a top view. FIG. 5(f) is a bottom view. As illustrated in FIG. 5, the contamination prevention box 9 includes an upper frame 7 and a lower frame 8. A screw groove is provided in an upper portion of the upper frame 7, and the upper frame 7 can be screw fitted (connected) with the lidded holder 2 by the screw groove. The connection method between the lidded holder 2 and the contamination prevention box 9 is not limited to the screw type. It is sufficient that the fixing strength that can withstand centrifugation by a centrifuge can be obtained. For example, the lidded holder 2 and the contamination prevention box 9 may be configured to be connectable simply by fitting.

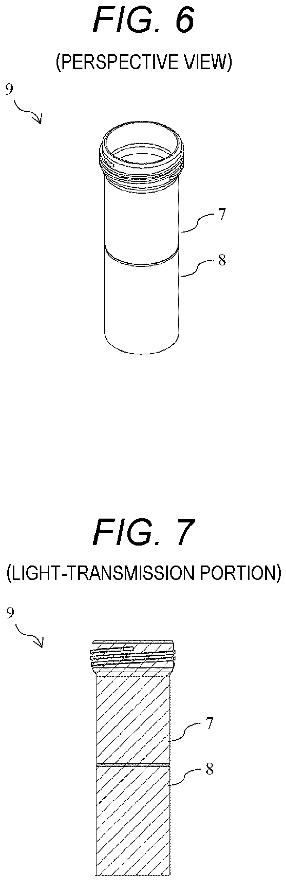

FIG. 6 is a perspective view of the contamination prevention box 9. As illustrated in FIG. 6, the contamination prevention box 9 has a substantially cylindrical shape.

FIG. 7 is a reference view illustrating a portion made of a light-transmissive material of the contamination prevention box 9. A hatched portion in FIG. 7, that is, the upper frame 7 and the lower frame 8 of the contamination prevention box 9 can be made of a material (resin or glass) that transmits light.

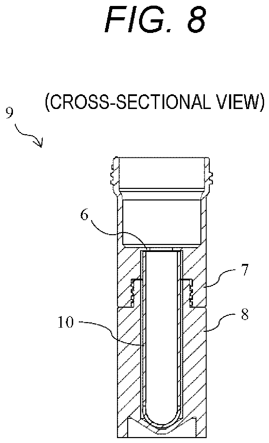

FIG. 8 is a cross-sectional view taken along line V-V of the contamination prevention box 9. As illustrated in FIG. 8, the upper frame 7 and the lower frame 8 are screwed and configured to be separable. As will be described later, a space capable of storing the filter cup 3 of the lidded holder 2 is provided in an upper portion of in internal space of the upper frame 7. A measurement tube 10 (measurement container) is provided inside the contamination prevention box 9. The measurement tube 10 is longer than the lower frame 8. When the upper frame 7 is removed from the lower frame 8, the measurement tube 10 protrudes from an opening surface of the lower frame 8.

A partition wall 6 having an opening is provided inside the upper frame 7. The partition wall 6 separates a space capable of storing the filter cup 3 and a space storing the measurement tube 10. A cross-sectional area of the opening of the partition wall 6 is smaller than a cross-sectional area of an opening of the measurement tube 10. The cross-sectional area of the opening of the partition wall 6 is larger than a cross-sectional area of the nozzle 5, and the nozzle 5 can pass through the opening of the partition wall 6.

### <Configuration Example of Second State of Centrifugal Filtration Cartridge>

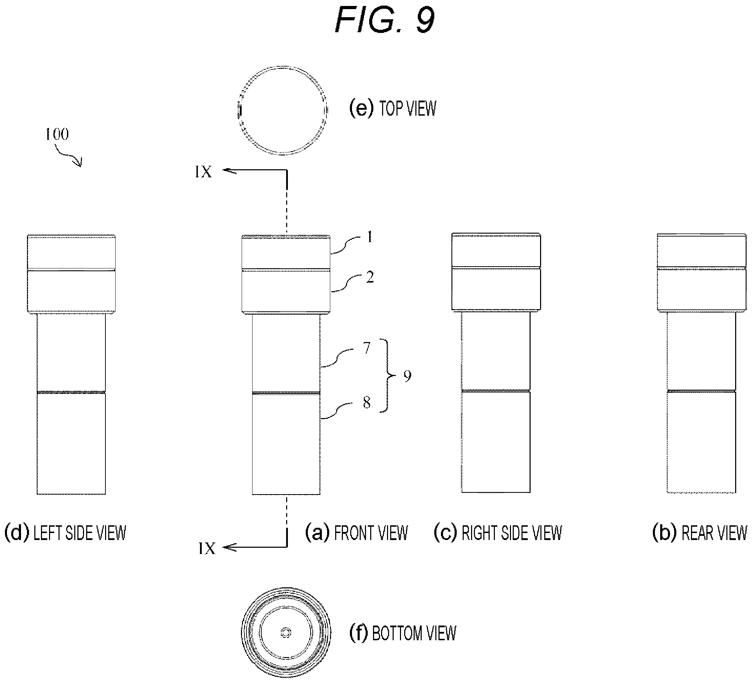

FIG. 9 is a six-sided view illustrating an appearance of the centrifugal filtration cartridge 100 according to the first embodiment in a second state. FIG. 9(a) is a front view. FIG. 9(b) is a rear view. FIG. 9(c) is a right side view. FIG. 9(d) is a left side view. FIG. 9(e) is a top view. FIG. 9(f) is a bottom view. As illustrated in FIG. 9, the centrifugal filtration cartridge 100 in the second state is in a state where the contamination prevention box 9 is connected to the lidded holder 2.

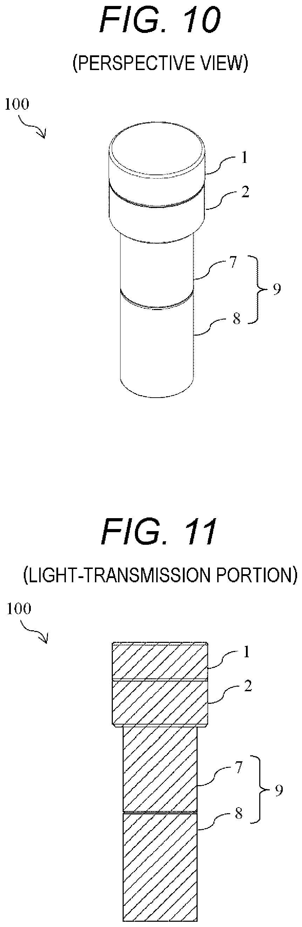

FIG. 10 is a perspective view of the centrifugal filtration cartridge 100 in the second state. As illustrated in FIG. 10, the lidded holder 2 and the contamination prevention box 9 have a substantially cylindrical shape. An outer diameter of the contamination prevention box 9 is smaller than the outer diameter of the lidded holder 2.

FIG. 11 is a reference view illustrating a portion made of a light-transmissive material of the centrifugal filtration cartridge 100 in the second state. A hatched portion in FIG. 11, that is, the lid 1, the lidded holder 2, and the contamination prevention box 9 can be made of a material (resin or glass) that transmits light.

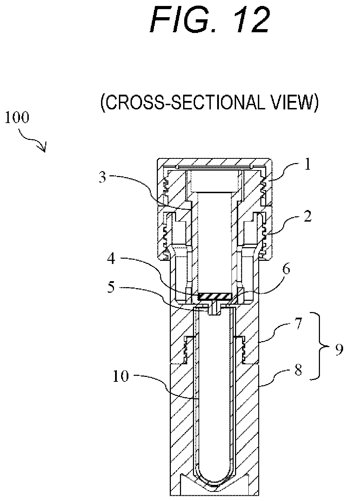

FIG. 12 is a cross-sectional view taken along line IX-IX of the centrifugal filtration cartridge 100 in the second state. As illustrated in FIG. 12, when the contamination prevention box 9 is connected to the lidded holder 2, the nozzle 5 enters inside of the measurement tube 10 through the opening of the partition wall 6. Consequently, the solution that has passed through the filter 4 and the nozzle 5 at the time of centrifugal filtration does not come into contact with a portion other than the inside of the measurement tube 10 of the centrifugal filtration cartridge 100. Therefore, contamination of the liquid introduced into the measurement tube 10 can be prevented. Since the measurement tube 10 is covered with the contamination prevention box 9, the measurement tube 10 is not exposed to the surrounding environment. Thus, it is possible to prevent adhesion of bacteria floating in the air, splashes from an operator, and the like. The nozzle 5 and the partition wall 6 are designed so that the nozzle 5 and the partition wall 6 do not come into contact with each other when the lidded holder 2 and the contamination prevention box 9 are connected. This can prevent the solution that has passed through the filter 4 from coming into contact with the partition wall 6.

The nozzle 5 may have a substantially cylindrical shape. The nozzle 5 may have a substantially rectangular cylindrical shape as well. A thickness of a tip of the nozzle 5 (diameter when cross-section is circular, maximum length of diagonal when cross-section is polygonal) can be, for example, 5 mm or less, and can be 3 mm or less depending on the case. This makes it possible to prevent the solution from remaining in the nozzle 5 at the time of centrifugal filtration using the centrifugal filtration cartridge 100.

The nozzle 5 can be designed such that a plane including a tip surface of the nozzle 5 is separated from a lower surface of the partition wall 6 by, for example, 1 mm or more, and 3 mm or more depending on the case. This makes it possible to prevent the solution from coming into contact with the contamination prevention box 9 and the partition wall 6 at the time of centrifugal filtration. The nozzle 5 can be designed such that the plane including the tip surface of the nozzle 5 is separated from an opening surface of the measurement tube 10 by, for example, 1 mm or more, and 5 mm or more depending on the case. This makes it possible to prevent the solution from leaking out of the measurement tube 10 at the time of centrifugal filtration.

### <Packaging of Centrifugal Filtration Cartridge>

The centrifugal filtration cartridge 100 can be distributed as a test kit including the lidded holder 2, the contamination prevention box 9, and the waste liquid container 12. In the test kit, the centrifugal filtration cartridge 100 in the first state where the lidded holder 2 and the waste liquid container 12 are connected and the contamination prevention box 9 alone can be sealed and packaged. By configuring the test kit in this manner, since replacement of the waste liquid container 12 and the contamination prevention box 9 only needs to be performed once at the time of use described later, the operation is simplified, and contamination from the outside can also be prevented. The lidded holder 2, the contamination prevention box 9, and the waste liquid container 12 can be packaged through sterilization processes such as gamma ray sterilization, EOG sterilization, and autoclaving. The centrifugal filtration cartridge 100 can be disposable.

### <Sterility Test Method Using Centrifugal Filtration Cartridge 100>

As an example of a usage pattern of the centrifugal filtration cartridge 100, a method of testing sterility of pharmaceutical water (test sample) using an ATP method will be described. In this method, a high-sensitive ATP test kit (Lumione (registered trademark) Reagent Kit for Rapid Microbial Assay (manufactured by Hitachi High-Tech Corporation)) and a high-sensitive ATP measurement device (Lumione BL-2000 (manufactured by Hitachi High-Tech Corporation) are used. The high-sensitive ATP test kit includes an ATP activity eliminating liquid, a washing liquid, a luminescent liquid, an extraction liquid, a positive control (ATP 100 amol/50 µL extract), a measurement tube, and a dedicated rack thereof.

The operation described below is performed in a clean room of grade A managed at less than 1 CFU/m³ or grade B managed at 10 CFU/m³ or less.

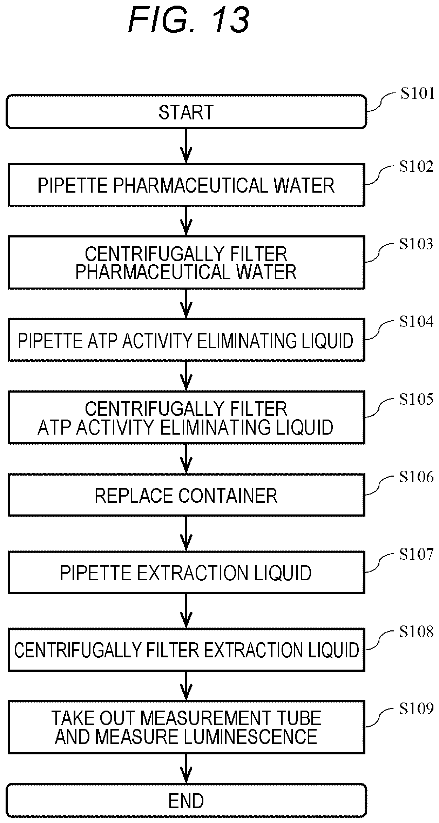

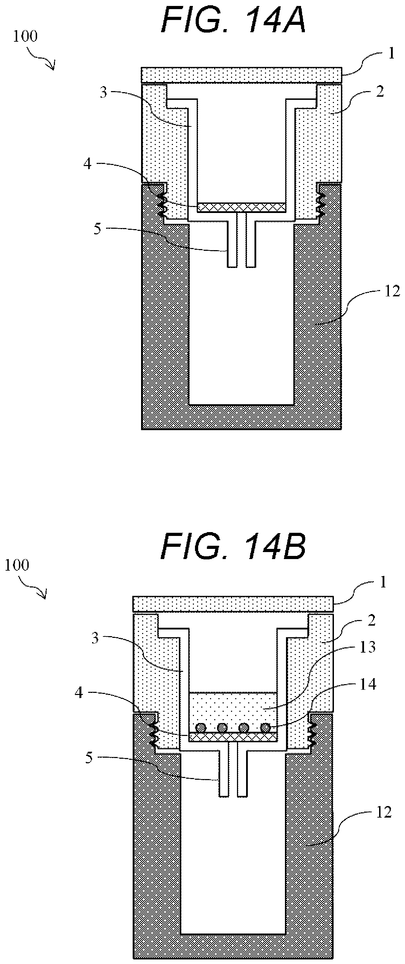

FIG. 13 is a flowchart illustrating a method of testing sterility of pharmaceutical water using the centrifugal filtration cartridge 100. FIGS. 14A to 14I are schematic cross-sectional views illustrating the state of the centrifugal filtration cartridge 100 in each step. In FIGS. 14A to 14I, for simplification of illustration, each member is illustrated with a scale, a shape, and a size different from those in FIGS. 4, 8, and 12.

### (Step S101: Initial state)

An operator prepares the centrifugal filtration cartridge 100, the high-sensitive ATP test kit, and the ATP measurement device. The operator sterilizes (for example, radiation sterilization, gas sterilization, or high-pressure steam sterilization) the centrifugal filtration cartridge 100 and all fixtures such as a pipette in advance. When the preparation is completed, the operator starts the operation of the sterility test.

FIG. 14A is a schematic cross-sectional view illustrating the centrifugal filtration cartridge 100 in the initial state (first state) in step S101. The filter 4 and the filter cup 3 are stored in the lidded holder 2. The waste liquid container 12 is connected to the lower portion of the lidded holder 2. The lid 1 of the lidded holder 2 is sealed.

### (Step S102: Pipetting of pharmaceutical water)

The operator opens the lid 1 of the lidded holder 2 and pipettes the pharmaceutical water (test sample) from a container containing the pharmaceutical water into the filter cup 3 by pipetting. Thereafter, the operator closes the lid 1. In order to smoothly perform centrifugal filtration, the volume of pharmaceutical water can be set to, for example, 100 milliliters or less, and 10 milliliters or less depending on the case.

FIG. 14B is a schematic cross-sectional view illustrating the state of the centrifugal filtration cartridge 100 in step S102. When pharmaceutical water 13 is contaminated, bacteria 14 are present in the pharmaceutical water 13. In the present embodiment, for clarity of description, a case where the bacteria 14 are present is illustrated.

### (Step S103: Centrifugal filtration of pharmaceutical water)

The operator sets the centrifugal filtration cartridge 100 in a centrifuge, and drives the centrifuge to centrifugally filter the pharmaceutical water 13.

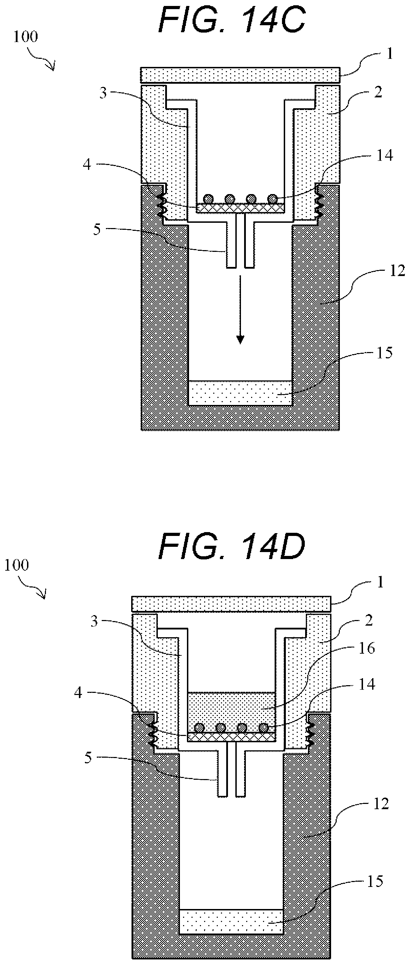

FIG. 14C is a schematic cross-sectional view illustrating the state of the centrifugal filtration cartridge 100 in step S103. The bacteria 14 contained in the pharmaceutical water 13 are trapped on the filter 4. A filtrate of the pharmaceutical water 13 is discharged as a waste liquid 15 to the waste liquid container 12.

### (Step S104: Pipetting of ATP activity eliminating liquid)

The operator opens the lid 1, pipettes the ATP activity eliminating liquid included in the high-sensitive ATP test kit onto the filter 4 of the filter cup 3, and closes the lid 1. By pipetting the ATP activity eliminating liquid onto the filter 4, unnecessary ATP contained in the test sample remaining in the filter cup 3 and the filter 4 can be erased. The volume of the ATP activity eliminating liquid can be larger than the volume of the pharmaceutical water 13 pipetted in step S102. After pipetting the ATP activity eliminating liquid, an eliminating reaction can be promoted by, for example, incubating at 37°C for 40 minutes or more.

FIG. 14D is a schematic cross-sectional view illustrating the state of the centrifugal filtration cartridge 100 in step S104. Even when an ATP activity eliminating liquid 16 is pipetted, ATP inside the bacterium 14 trapped on the filter 4 is not erased by the ATP activity eliminating liquid 16 because ATP is covered by a cell membrane and a cell wall. Thus, the ATP activity eliminating liquid 16 can erase ATP other than those derived from a bacterium.

### (Step S105: Centrifugal filtration of ATP activity eliminating liquid)

The operator sets the centrifugal filtration cartridge 100 in the centrifuge, and drives the centrifuge to centrifugally filter the ATP activity eliminating liquid 16.

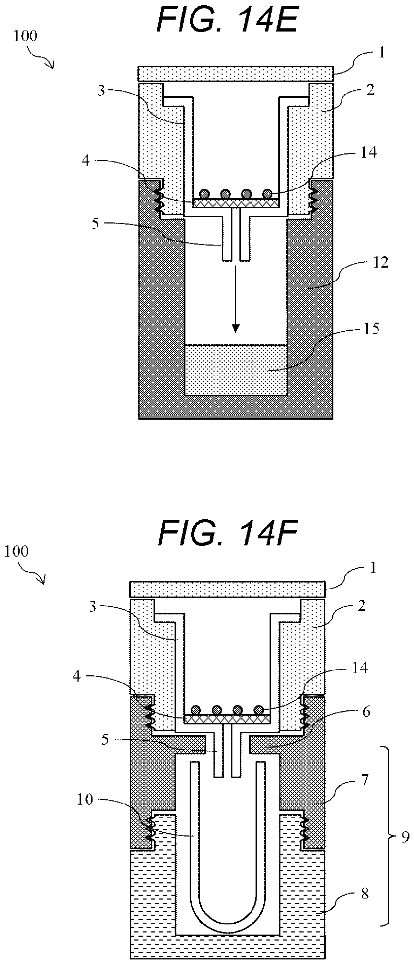

FIG. 14E is a schematic cross-sectional view illustrating the state of the centrifugal filtration cartridge 100 in step S105. The ATP activity eliminating liquid 16 is discharged as the waste liquid 15 to the waste liquid container 12 below the filter.

### (Step S106: Container replacement)

The operator removes the waste liquid container 12 from the lidded holder 2 and attaches the contamination prevention box 9 in which the measurement tube 10 is stored to the lidded holder 2.

FIG. 14F is a schematic cross-sectional view illustrating the state of the centrifugal filtration cartridge 100 in step S106. The waste liquid container 12 is removed from the lidded holder 2, and the contamination prevention box 9 in which the measurement tube 10 is stored is set in the lidded holder 2. The tip of the nozzle 5 provided below the filter 4 passes through the opening of the partition wall 6 and enters the inside of the measurement tube 10.

### (Step S107: Pipetting of extraction liquid)

The operator opens the lid 1, pipettes the extraction liquid included in the high-sensitive ATP test kit onto the filter 4 of the filter cup 3, and closes the lid 1. The volume of the extraction liquid can be set to, for example, 500 µL or less, and 50 µL or less depending on the case. The extraction liquid dissolves the cell membrane and the cell wall of the bacterium 14 trapped on the filter 4, and releases ATP inside the bacterium 14 into the extraction liquid. ATP extraction can be sufficiently performed, for example, by allowing the extraction liquid to stand for 1 minute or more after pipetting the extraction liquid.

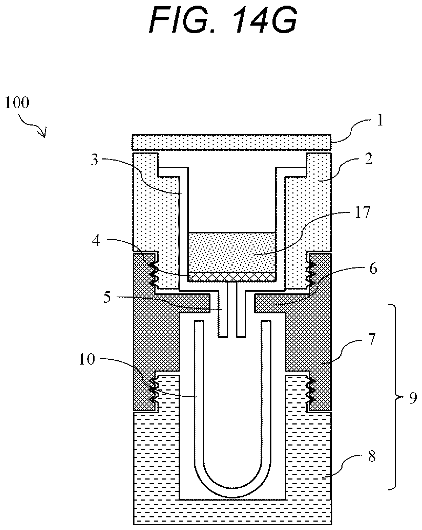

FIG. 14G is a schematic cross-sectional view illustrating the state of the centrifugal filtration cartridge 100 in step S107. When an extraction liquid 17 is pipetted, the cell membrane and the cell wall of the bacterium 14 are destroyed, and ATP inside the bacterium 14 is dissolved in the extraction liquid 17.

### (Step S108: Centrifugal filtration of extraction liquid)

The operator sets the centrifugal filtration cartridge 100 in the centrifuge, and drives the centrifuge to centrifugally filter the extraction liquid 17 in which ATP derived from the bacterium 14 is dissolved.

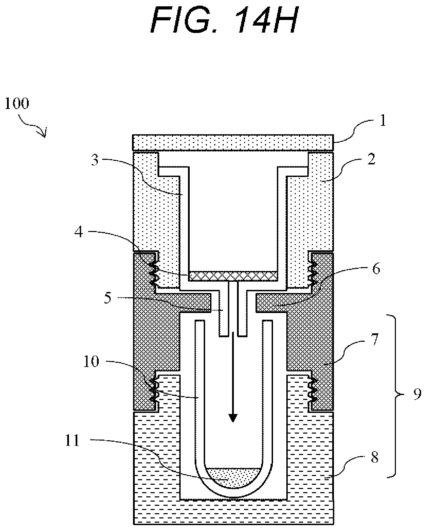

FIG. 14H is a schematic cross-sectional view illustrating the state of the centrifugal filtration cartridge 100 in step S108. The extraction liquid 17 containing ATP derived from the bacterium 14 passes through the filter 4 and the nozzle 5 and is delivered to the measurement tube 10. The filtrate collected in the measurement tube 10 is used as a specimen 11 for luminescence measurement described later. Since the tip of the nozzle 5 enters the inside of the measurement tube 10, the filtrate to be the specimen 11 does not leak out of the measurement tube 10. Since the periphery of the measurement tube 10 is covered with the contamination prevention box 9 except for the minimum opening, the risk of contamination during operation can be reduced as compared with a conventional case where the measurement tube 10 is exposed.

### (Step S109: Taking out of measurement tube and measuring luminescence)

The operator removes the lower frame 8 of the contamination prevention box 9 from the upper frame 7 and takes out the measurement tube 10 from the lower frame 8. The measurement tube 10 can be taken out in a clean environment such as the inside of a safety cabinet. After taking out the measurement tube 10, the operator sets the measurement tube 10 in the ATP measurement device, and measures an amount of ATP bioluminescence derived from a bacterium by the ATP measurement device. When the amount of luminescence exceeds a threshold value adopted in advance, it indicates that bacteria with a value greater than or equal to a specified value have been present in the test sample (pharmaceutical water).

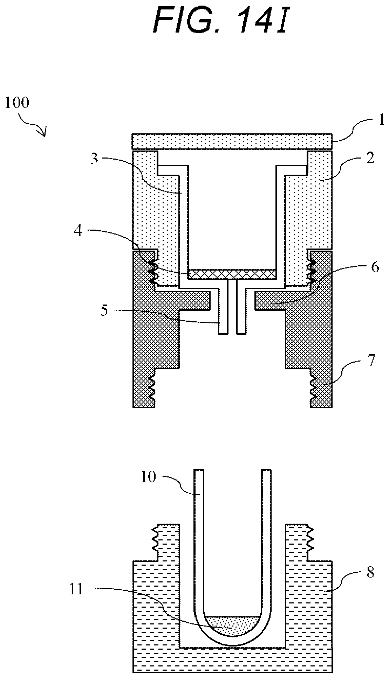

FIG. 14I is a schematic cross-sectional view illustrating the state of the centrifugal filtration cartridge 100 in step S109. FIG. 14I illustrates a state where the lower frame 8 of the contamination prevention box 9 is removed from the upper frame 7. Since an upper portion of the measurement tube 10 protrudes from the lower frame 8, the operator can easily grip the measurement tube 10. An amount of protrusion of the measurement tube 10 from the lower frame 8 can be set to, for example, such a length that when the operator grips the measurement tube 10, the finger does not come into contact with the opening of the measurement tube 10. Specifically, the amount of protrusion of the measurement tube 10 from the lower frame 8 can be, for example, 2 cm or more.

### <Type of Test Sample>

The sterility test method when the test sample is pharmaceutical water has been described above. The centrifugal filtration cartridge 100 of the present disclosure can be applied to other than centrifugal filtration of pharmaceutical water. For example, a beverage product or pharmaceutical product (for example, internal medicine, external medicine, injection, cell therapeutic agent, and gene therapeutic agent) can be the test sample. Therefore, the centrifugal filtration cartridge 100 can be used for in-process inspection or pre-shipment inspection of a beverage product, in-process inspection or pre-shipment inspection of a medicine, or the like.

### <Summary of First Embodiment>

As described above, the centrifugal filtration cartridge 100 according to the first embodiment includes the filter cup 3 and the contamination prevention box 9. The filter cup 3 includes the filter 4 that traps microorganisms and the nozzle 5 that discharges the liquid having passed through the filter 4. The contamination prevention box 9 is configured to be connectable to the filter cup 3 and internally stores the measurement tube 10 capable of storing the liquid discharged from the nozzle 5. The contamination prevention box 9 has the partition wall 6 disposed between the filter cup 3 and the opening surface of the measurement tube 10. The partition wall 6 has the opening through which the nozzle 5 can pass. The nozzle 5 has a length in which the tip of the nozzle 5 is located below a lower end of the opening and enters the inside of the measurement tube 10 when the contamination prevention box 9 is connected to the filter cup.

As described above, the measurement tube 10 is stored in the contamination prevention box 9 and the partition wall 6 is present between the opening surface of the measurement tube 10 and the filter cup 3. This allows contamination of the measurement tube 10 to be reduced from the surrounding environment. The tip of the nozzle 5 enters the inside of the measurement tube 10 (the tip is located below the opening surface). Consequently, the entire amount of the liquid (specimen that may contain the target substance) passing through the filter 4 can be accurately moved to the measurement tube 10 by centrifugal filtration without coming into contact with other components than the filter 4, the nozzle 5, and the measurement tube 10. As a result, it is possible to reduce false positives and false negatives in the microbial test.

### [Second Embodiment]

In the first embodiment described above, the centrifugal filtration cartridge capable of testing one test sample at a time has been described. However, depending on the test, it is desired that the same test sample is divided into a plurality of pieces and tested a plurality of times. For example, since it is difficult to determine false positive/false negative in one test, it is conceivable to perform measurement a plurality of times and determine as positive as a test result when positive results are obtained a predetermined number of times or more. Thus, in the second embodiment, a centrifugal filtration cartridge capable of acquiring a specimen from one test sample to a plurality of measurement tubes is proposed.

### <Configuration Example of Centrifugal Filtration Cartridge>

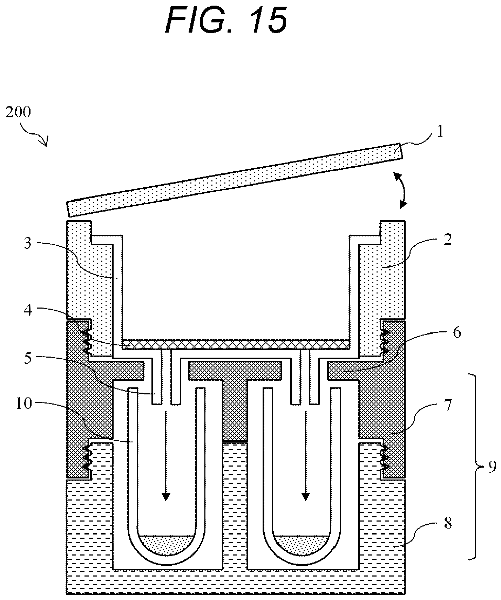

FIG. 15 is a schematic cross-sectional view illustrating a centrifugal filtration cartridge 200 according to the second embodiment. FIG. 15 illustrates a state where a contamination prevention box 9 is connected to a lidded holder 2. The centrifugal filtration cartridge 200 differs from the centrifugal filtration cartridge 100 of the first embodiment mainly in that a filter cup 3 has two nozzles 5 and the contamination prevention box 9 stores two measurement tubes 10. The spaces for storing the two measurement tubes 10 are separated from each other. A partition wall 6 is provided with an opening at each of positions corresponding to the two nozzles 5. Each opening of the partition wall 6 is larger than the cross-sectional area of the nozzle 5 and smaller than an area of an opening of the measurement tube 10. The number of the nozzles 5, the number of the openings of the partition wall 6, and the number of the measurement tubes 10 are not limited to two, and may be three or more.

In the present embodiment, a lid 1 is configured to be openable and closable by a hinge mechanism, but may be of a screw type as in the first embodiment.

Although not illustrated, the waste liquid container 12 connected to the lidded holder 2 is not necessarily separated into two spaces, but may be separated into two spaces.

### <Sterility Test Method Using Centrifugal Filtration Cartridge>

Since the sterility test method of the present embodiment can be similar to that of the first embodiment, the description thereof will be omitted. However, according to the present embodiment, the specimen is collected in the two measurement tubes 10 in step S108 described above. Thereby, ATP luminescence measurement can be performed simultaneously for the two specimens.

### <Summary of Second Embodiment>

As described above, the contamination prevention box of the centrifugal filtration cartridge according to the second embodiment includes the plurality of measurement tubes stored in different spaces. Consequently, since a plurality of specimens can be acquired from one test sample, accuracy of the test result can be improved.

### [Third Embodiment]

In the first embodiment, in order to reduce contamination of the measurement tube 10 from the surrounding environment, the centrifugal filtration cartridge 100 that stores the measurement tube 10 in the contamination prevention box 9 has been described. The structure of the first embodiment can sufficiently reduce mixing of an inhibitory substance. The third embodiment proposes a centrifugal filtration cartridge capable of achieving more thorough sealability.

### <Configuration Example of Centrifugal Filtration Cartridge>

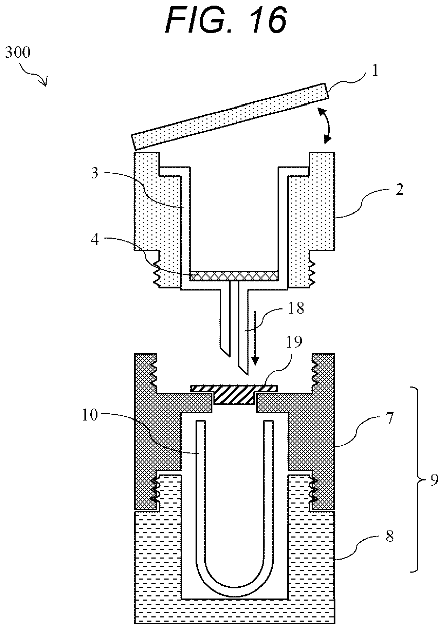

FIG. 16 is a schematic cross-sectional view illustrating a centrifugal filtration cartridge 300 according to the third embodiment. FIG. 16 illustrates a state immediately before the contamination prevention box 9 is connected to a lidded holder 2. The centrifugal filtration cartridge 300 is different from the centrifugal filtration cartridge 100 of the first embodiment in that a hermetic seal 19 is provided in an opening of a partition wall 6 of the contamination prevention box 9, and a tip of a nozzle 18 is processed into a needle shape to penetrate the hermetic seal 19.

As the hermetic seal 19, for example, a rubber-like septum, an aluminum foil film, or the like can be used. When the contamination prevention box 9 is connected to the lidded holder 2, the operator pierces the hermetic seal 19 with the nozzle 18 to cause the nozzle 18 to enter the measurement tube 10.

In the state where the contamination prevention box 9 is connected to the lidded holder 2, the nozzle 18 can be designed such that a plane including the tip of the nozzle 18 is separated from a lower surface of the partition wall 6 by, for example, 1 mm or more, and 3 mm or more depending on the case. This makes it possible to prevent the solution from coming into contact with the contamination prevention box 9 and the partition wall 6 at the time of centrifugal filtration. The nozzle 18 can be designed such that the plane including the tip of the nozzle 18 is separated from an opening surface of the measurement tube 10 by, for example, 1 mm or more, and 5 mm or more depending on the case. This makes it possible to prevent the solution from leaking out of the measurement tube 10 at the time of centrifugal filtration.

### <Sterility Test Method Using Centrifugal Filtration Cartridge>

Since the sterility test method of the present embodiment can be similar to that of the first embodiment, the description thereof will be omitted.

### <Summary of Third Embodiment>

As described above, in the contamination prevention box 9 of the centrifugal filtration cartridge 300 according to the third embodiment, the hermetic seal 19 is provided in the opening of the partition wall 6. As a result, the sealability and sterility of the measurement tube 10 are maintained until the contamination prevention box 9 is connected to the lidded holder 2. Thereby it is possible to more reliably prevent contamination of the measurement tube 10.

### [Modifications]

The present disclosure is not limited to the above embodiments, and includes various modifications. For example, the above embodiments have been described in detail for easy understanding of the present disclosure, and the present disclosure does not necessarily have all the configurations described. In addition, some of certain embodiment can be replaced with the configuration of the other embodiment. Further, it is possible to add the configuration of one embodiment to the configuration of another embodiment. It is also possible to add, delete, or replace a part of the configuration of another embodiment with respect to a part of the configuration of each embodiment.

### Reference Signs List

- 1: lid
- 2: lidded holder
- 3: filter cup
- 4: filter
- 5: nozzle
- 6: partition wall
- 7: upper frame (first box)
- 8: lower frame (second box)
- 9: contamination prevention box
- 10: measurement tube (measurement container)
- 11: specimen
- 12: waste liquid container
- 13: pharmaceutical water
- 14: bacterium (microorganism)
- 15: waste liquid
- 16: ATP activity eliminating liquid
- 17: extraction liquid
- 18: nozzle
- 19: hermetic seal
- 100 to 300: centrifugal filtration cartridge

## Claims

1. A centrifugal filtration cartridge comprising:
a filter cup including a filter that captures a microorganism and a nozzle that discharges a liquid that has passed through the filter; and
a contamination prevention box connectable to the filter cup and internally storing a measurement container configured to store the liquid discharged from the nozzle, wherein
the contamination prevention box includes a partition wall disposed between the filter cup and an opening surface of the measurement container,
the partition wall has an opening through which the nozzle can pass, and
the nozzle has a length in which a tip of the nozzle is located below a lower end of the opening and enters inside of the measurement container when the contamination prevention box is connected to the filter cup.

2. The centrifugal filtration cartridge according to claim 1, wherein a cross-sectional area of the opening is larger than a cross-sectional area of the nozzle and smaller than a cross-sectional area of the opening surface of the measurement container.

3. The centrifugal filtration cartridge according to claim 1, wherein
the contamination prevention box includes a first box on an opening surface side of the measurement container and a second box on a bottom side of the measurement container, and
the first box and the second box are configured to be separable.

4. The centrifugal filtration cartridge according to claim 1, wherein
the contamination prevention box stores a plurality of the measurement containers,
the partition wall has a plurality of the openings provided in respective upper portions of the plurality of measurement containers, and
the filter cup has a plurality of the nozzles equal in number to the number of the plurality of measurement containers.

5. The centrifugal filtration cartridge according to claim 1, wherein
the opening includes a seal that seals the opening, and
the tip of the nozzle has a needle shape.

6. The centrifugal filtration cartridge according to claim 1, wherein the nozzle has a thickness of 5 mm or less.

7. The centrifugal filtration cartridge according to claim 1, wherein, in a state where the contamination prevention box is connected to the filter cup, a plane including the tip of the nozzle is located at a distance of 1 mm or more below from a lower surface of the partition wall.

8. The centrifugal filtration cartridge according to claim 1, wherein, in a state where the contamination prevention box is connected to the filter cup, a plane including the tip of the nozzle is located at a distance of 1 mm or more below from the opening surface of the measurement container.

9. The centrifugal filtration cartridge according to claim 1, wherein there is a gap between a side surface of the nozzle and the opening in a state where the contamination prevention box is connected to the filter cup.

10. The centrifugal filtration cartridge according to claim 1, further comprising a holder that holds the filter cup, has a lid covering an upper side of the filter cup, and is connectable to the contamination prevention box.

11. The centrifugal filtration cartridge according to claim 3, wherein the measurement container protrudes from the second box when the first box and the second box are separated.

12. The centrifugal filtration cartridge according to claim 1, further comprising a waste liquid container connectable to the filter cup in a state where the contamination prevention box is removed therefrom.

13. A microbial test method comprising:
preparing the centrifugal filtration cartridge according to claim 1;
pipetting a test sample into the filter cup;
connecting the filter cup and the measurement container; and
installing the centrifugal filtration cartridge in a centrifuge in a state where the filter cup and the measurement container are connected to each other, and performing centrifugal filtration with the filter.
